# EUROPEAN PATENT APPLICATION

(11) **EP 2 545 944 A1**
(43) Date of publication of application: **16.01.2013**
(21) Application number: 11752903.2
(22) Date of filing: 11.03.2011
(51) Int. Cl.: A61L 24/02, A61K 6/06

(54) **INORGANIC CEMENT FOR BIOMEDICAL USES, PREPARATION METHOD THEREOF AND USE OF SAME**

(30) Priority: 12.03.2010 ES 201000359
(71) Applicant: Universitat Politécnica De Catalunya, 08034 Barcelona (ES)
(72) Inventor: GINEBRA MOLINS, Maria Pau, E-08034 Barcelona (ES); MESTRES BEÀ, Gemma, E-08034 Barcelona (ES)
(74) Representative: Juncosa Miro, Jaime
(86) International application number: PCT/ES2011/070170
(87) International publication number: WO 2011/110724

(57) **Abstract**

The present invention relates to a cement based on magnesium sodium phosphate with clinical applications in bone surgery and odontology. This cement has an intrinsic antimicrobial effect as its main property. It also has a fast setting time, along with a high short-term compressive strength. Another property inherent to these cements is their adhesive character.

Use of the cement is indicated for bone and dental applications, as well as filling bone defects or sealing dental cavities. The cement is especially indicated in cases in which an antimicrobial effect and/or adhesive properties are necessary.

## Description

### Field of the Invention

The present invention relates to biomaterials for regenerating hard tissues: bone surgery and odontology. These materials can be prepared in the form of granules, cements, coverings, dense or porous ceramics, etc. They can be applied for filling bone cavities, stabilising bone fractures and covering prostheses or implants.

The cement of the present invention is obtained from magnesium oxide and a sodium phosphate and is especially indicated in cases where an antimicrobial effect is necessary, for example, in infections associated with metal prostheses (peri-implantitis). On the other hand, its adhesive properties make it useful for fixing dental implants or prostheses, in treating periapical diseases or, generally, for sealing in endodontic treatments. They can also act as drug delivery systems and tissue engineering scaffolds. The present invention also relates to methods for obtaining said biomaterials.

This invention also relates to a method for preparing the mentioned cement and to a use of said cement for bone and dental applications.

### State of the Prior Art

From the mid-eighties, the scientific community has made significant progress in the field of designing and manufacturing new materials for replacing and regenerating bone tissue. One type of materials which has been widely studied is that of materials based on calcium phosphate. Among the different forms in which calcium phosphates can be used, cements deserve special attention due to certain intrinsic advantages such as their workability and perfect adaptation to the bone cavity, the possibility of injecting them using minimally invasive techniques and the fact that they are transformed within the body giving rise to hydroxyapatite or another calcium phosphate, in turn causing the cement to set. It is of interest to highlight that hydroxyapatite which can be obtained as setting product from calcium phosphate cement has numerous similarities with the natural inorganic bone component, such as the chemical composition and the nanometric size of the crystals [1].

Despite the renowned capacity of apatite cements to satisfactorily regenerate damaged bone, they also have certain limitations. On one hand, the setting and hardening reaction is slow; on the other hand, their mechanical properties are poor, having a high fragility [1].

In an environment far removed from that of biomaterials, i.e., in the fields of civil engineering and construction, many studies performed during the last 30 years have led to the development of cements based on magnesium phosphate. These cements are obtained from magnesium oxide (MgO) and ammonium dihydrogen phosphate (NHₐH₂PO₄), producing struvite (MgNH₄PO₄·6H₂O), a magnesium ammonium phosphate, as a final reaction product. Currently they are materials widely used in the construction field, which are characterised by a high setting speed, and a high mechanical strength [2-4].

It should be pointed out that in the field corresponding to biomedicine there are very few studies analysing the viability of using cements based on magnesium phosphates. Wu *et al.* proposed preparing a magnesium phosphate cement based on magnesium oxide and ammonium dihydrogen phosphate, and its combination with calcium phosphate cements in different ratios. *In vitro* studies showed that the set cements were biocompatible. The *in living* implanting of a cement based on a mixture of magnesium phosphate and calcium phosphate also showed that said material was not toxic [5]. However, the fact that one of the reagents used was ammonium dihydrogen phosphate, a possible releaser of ammonia and/or ammonium ions in the surrounding medium [6], does not seem to be optimum from the point of view of the material innocuousness or possible side effects. It should be pointed out that patent US 7,094,286 B2, proposes preparing magnesium phosphate cements using monocalcium phosphate monohydrate or monocalcium phosphate anhydrous as a possible source of phosphate in addition to ammonium phosphate or ammonium polyphosphate, and further mixing it with calcium phosphate cement [7].

In Patents US 7,115,163 B2 and US 6,692,563 B2, Zimmermann also describes a cement which after setting consists of a mixture of magnesium ammonium phosphate and calcium phosphate (despite the fact that only magnesium ammonium phosphate cement is referred to in the patent titles). Said material is presented as being biologically degradable and with adhesive properties [8,9]. Finally, patent US 6,533,821 B1, Lally, proposes a bioadhesive consisting of mixing potassium phosphate, a metal oxide, a calcium-containing compound and water [10].

In a considerably different approach, Klammert *et al.* have recently developed a calcium magnesium phosphate cement formed from the reaction of magnesium-substituted tricalcium phosphate with monocalcium phosphate monohydrate, a mixture of brushite and newberyite being obtained as the reaction product. Said cement has an increase of mechanical properties and greater cellular proliferation [11] as the most significant results.

### Brief Description of the Invention

Unlike the mentioned papers and patent applications, this invention proposes an inorganic cement based on magnesium oxide and a sodium phosphate for clinical, bone or dental applications. Said cement has several intrinsic properties making it of special interest. The first feature of the material to highlight is that it increases the pH of its immediate environment, a property giving it an antimicrobial effect, as has previously been found in other materials [12-14]. Furthermore, the active oxygen released by one of the components present in the cement, magnesium oxide, reinforces said effect [15]. These antimicrobial properties make the developed cement an ideal candidate for some clinical applications where an infectious process must be controlled, as in the case of infections associated with bone or dental implants (peri-implantitis), or certain pulp or periapical diseases, among other applications.

In addition to antimicrobial properties, the developed cement has high mechanical strength and fast setting, giving it a high short-term strength, a common feature in other magnesium phosphate cements. These properties give the material the capacity to withstand loads shortly after it is implanted, unlike calcium phosphate cements. Furthermore, they also have a high capacity of adhesion to living materials or objects.

The composition of the material has been optimised for the purpose of assuring good biocompatibility, specifically in two aspects: i) the formulation of the cement has been adjusted to reduce the exothermic nature of the setting reaction for the purpose of preventing tissue necrosis, ii) using sodium phosphate as a phosphate source is proposed, whereby the release of toxic by-products is prevented or reduced, the release of which products can occur in magnesium cements containing ammonium phosphate [6,16].

Preparing such cement basically requires two types of components: a) an oxide, in the present case magnesium oxide and b) one or more compounds containing phosphates, in the present invention sodium phosphate. A setting reaction retarder can further be added to extend the setting time and, in turn, dampen the exothermic reaction occurring when mixing the reagents. There are also other parameters which allow controlling the heat released during the setting reaction, such as the reactivity of the oxide, the reagent particle size and the liquid/powder ratio of the cement.

Finally, it is important to point out that the reaction product is amorphous magnesium sodium phosphate, unlike the product formed when ammonium phosphate is used as a reagent, in which case struvite, a crystalline compound [16], is mainly formed.

### Detailed Description of the Invention

This invention presents an inorganic cement for bone and/or dental applications, comprising a mixture of a solid phase formed by magnesium oxide (MgO) and a sodium phosphate, and a liquid phase formed by water or an aqueous solution. The cement in question has several specific properties making it of special interest. For the purpose of enhancing said properties, the preparation conditions of the material have been optimised as detailed below.

A first aspect of this invention is that the cement has an intrinsic antimicrobial effect. This effect is due to the composition of the cement. On one hand, the pH increase occurring in the medium surrounding the cement [12-14] and, on the other hand, the active oxygen which is released in the medium due to the presence of an excess of magnesium oxide [15] are the mechanisms through which said effect takes place. Due to the reasons explained, among others which will be explained below, magnesium oxide is added at a molar amount greater than that of phosphate salt, such that the antimicrobial properties of the cement are enhanced. Said antimicrobial properties are of special interest since they make the cement a very suitable candidate for some treatments in orthopaedic surgery and in endodontic surgery, among other possible applications.

A second aspect of this invention is that said cement sets fast, with a high short-term compressive strength. There are several parameters affecting the mechanical properties and setting time, such as the ratio of the oxide with respect to the compound containing phosphates, the reactivity of the oxide, the reagents used, the liquid/powder ratio for preparing the cement and the amount of retarder added.

A third aspect of this invention, essential so that this material can be used in clinical applications, is that it is biocompatible. To that end it is necessary that: i) during the setting reaction the cement does not reach temperatures greater thane 40-45°C, representing a threshold above which the protein denaturation and surrounding tissue necrosis can start, and ii) toxic by-products are not released. Both conditions can be controlled, respectively, by means of i) controlling the setting reaction kinetics, for example, decreasing the reactivity of the reagents, increasing the particle size thereof and/or adding a retarder to the reaction; ii) selecting reagents not containing chemical elements or compounds susceptible to having harmful effects, such as ammonium phosphate present in magnesium ammonium phosphate cements [6-9].

A fourth aspect of this invention is the formation of an amorphous reaction product, unlike the crystalline product obtained in cements based on magnesium oxide and ammonium phosphate, in which struvite [16] is formed.

A fifth aspect of this invention is the high adhesion which the cement has to living materials or objects. The method of preparing the proposed cement requires two components. On one hand, a magnesium oxide favouring a high basicity of the final material and, on the other hand a compound containing phosphates which is slightly acidic. A retarder can also be added, which makes the setting reaction slower and thus allows reducing the exothermic nature of the process caused by the reaction between the basic component and the acid. Given that the heat released during setting could be detrimental to the surrounding tissue, reducing the exothermic nature of the reaction is a key aspect of this invention. In addition to adding a retarder, the exothermic nature can also be reduced by decreasing the reactivity of the reagents, increasing the particle size thereof and/or increasing the liquid/powder ratio of the cement. The more retarder is added to the cement powder, the greater the setting time is and the lower the temperature which the cement reaches when setting is.

The recommended reagents for preparing magnesium sodium phosphate cements are indicated below. The basic compound provides the magnesium, and the preferred option is magnesium oxide (MgO).

The use of sodium phosphate salt is proposed as a phosphate source, sodium dihydrogen phosphate (NaH₂PO₄) being the preferred reagent. Sodium dihydrogen phosphate can also be combined with another phosphate such as NH₄H₂PO₄, in different molar ratios.

The possible compounds which can be used as retarders are sodium fluorosilicate, sodium polyphosphate, sodium borate, boric acid, boric acid ester, and mixtures derived therefrom. Among those mentioned, sodium borate decahydrate or borax (Na₂B₄O₇·10 H₂O) is preferred. The retarder can be added to the cement powder in the solid phase or be dissolved in the liquid phase of the cement.

To favour the formation of the reaction product [17], as well as for enhancing a greater antimicrobial effect, it is recommended that magnesium oxide is at a greater molar amount than sodium phosphate. The recommended magnesium oxide to sodium phosphate molar ratio is in the 1<MgO:NaH2PO4<6 range, the 3<MgO:NaH2PO4<5 range being preferred. The amount of retarder added regulates the setting time, as well as the short-term mechanical properties and the exothermic nature of the reaction. The amount of retarder to be added can range between 0.05-10% by weight. Preferably, an amount of retarder between 2-6% by weight is used.

For the purpose of achieving suitable setting of the cement, as well as a moderate exothermic nature, controlling the reagent particle size is necessary. In the case of MgO, reducing its reactivity to control the exothermic nature is also necessary. The reactivity of MgO can be reduced by means of calcining thereof, which can be performed at a temperature greater than 1400°C for 0.5-15h. MgO particle size can range between 0.1-100 µm. If necessary, MgO can be ground to reduce its particle size. Phosphate reagents require a particle size between 50-500 µm to assure a good mixture with the alkaline compound, but without causing a reaction which is too exothermic. If necessary, the phosphate reagent can be ground. For better retarder efficiency, it is convenient that the retarder also has a particle size between 50-500 µm to assure a good mixture with the majority reagents. If necessary, the retarder can be ground.

In reference to the first aspect of the invention, the antimicrobial effect of the magnesium sodium phosphate cement has been demonstrated in the present patent due to two complementary effects. In other studies it has been shown that when the pH of the surrounding medium has a value greater than 9.5, this is toxic for most microbes [12]. The effect of the pH can also be enhanced by means of changes in the ratios of the reagents used, as well as in their particle size distribution and reactivity. For example, the increase in MgO reactivity by means of calcination at a lower temperature, or the increase of the sodium phosphate particle size by means of a less vigorous grinding, are two methods for increasing the pH produced by the cement and thus increasing the antimicrobial effect. Finally, it has been reported that MgO can release active oxygen, a component which also has a toxic effect for microbes [15].

Magnesium sodium phosphate cement is of interest for bone and dental applications, especially in situations where a microbial infection is to be fought. Said cement can be used in endodontic therapies. It can be applied for directly or indirectly covering pulp, for example, in the case of deep caries. It can also be used for sealing dentinal tubules and in treating pulpal or periapical inflammations, and in apexification therapies, consisted of filling the pulp with a material inducing the development of the root or the closure of the end thereof. The cement could also be used to fill the root canal in endodontic therapy. The antimicrobial properties of the cement reduce the risk of pulpal and root area re-infection.

### Preparation of the magnesium phosphate cement

The powder for preparing the magnesium phosphate cement is obtained by homogenising the powders of the compounds indicated in the recommended ratios.

The preparation of the cement paste requires adding an aqueous liquid, preferably water. This liquid can be added at a liquid/powder ratio of 0.05-0.30 mL/g, preferably 0.10-0.20 mL/g.

Obtaining a paste with a good consistency requires constantly stirring the powder with the liquid for a time between 45 s and 2 min. When this time has passed, a paste with a workable consistency which is ready for being introduced into a dental or bone cavity is obtained.

It is important to take into account that to use the cement *in vivo,* the powder and liquid must have been sterilised beforehand. Furthermore, a clean and, if possible, sterile working area is required to prevent contaminating the reagents at the time of preparing and introducing the paste.

### Preferred Embodiments

The examples included below are described for the purpose of illustrating the specific compositions described therein and the method of preparing the mixture of ingredients in a practical and non-limiting manner.

### Example 1: Magnesium phosphate cement prepared based on magnesium oxide, sodium hydrogen phosphate and borax

50 g of MgO calcined at 1475°C for 6 h were ground in an agate jar by means of using 4 agate balls, a planetary mill and grinding conditions of 150 rpm for 15 min. The same process was performed separately for NaH₂PO₄ and borax. 25 g of MgO were mixed with 19.584 g of NaH₂PO₄ and 1.379 g of borax in a homogeniser for 20 min. The preparation of the paste consisted of mixing 1.5 g of powder with 195 µL of water, and it was homogenised for 1 min to yield a paste with good consistency.

The initial and final setting time were measured by means of the Gillmore needle test [18], according to which the initial setting time is defined as the time taken from the moment the powder contacts the liquid until a pressure of 0.3 MPa leaves no mark on the surface of the cement; and the final setting time as the time elapsed from the moment the powder contacts the liquid until a pressure of 5 MPa leaves no mark on the surface of the cement. The test was performed by introducing the cement paste into plastic cylindrical moulds 10 mm in height. The initial setting time of the cement was 8 min, and the final time was 9 min. The exothermic nature of the cement was evaluated by inserting the tip of a thermocouple into the recently prepared paste. The temperature of the cement was monitored until it reached its maximum value. The maximum temperature reached during the setting of the cement was 41.3°C.

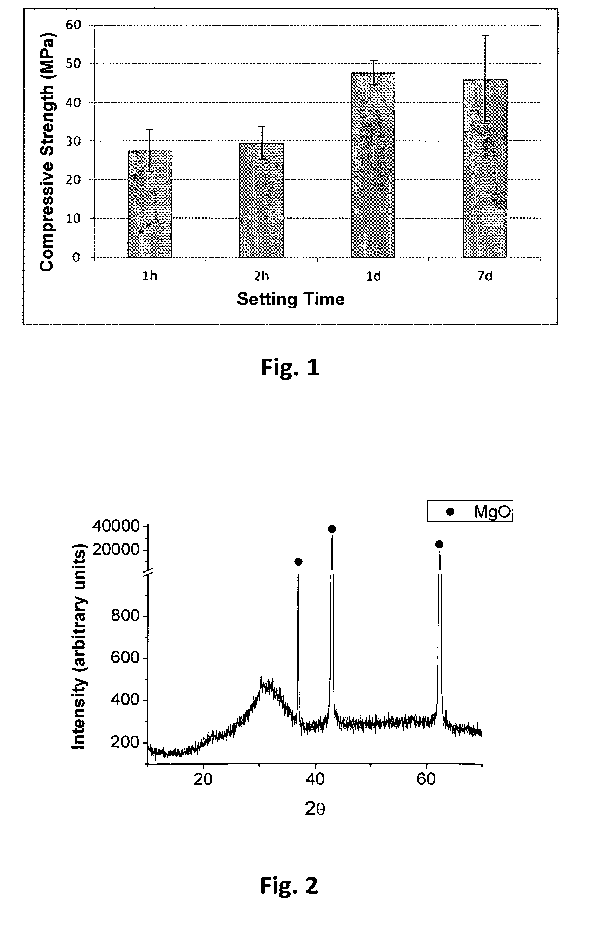

Test specimens of the cement 12 mm in height and 6 mm in diameter were prepared in Teflon moulds to measure compressive strength. Said moulds were introduced in a 0.9% wt NaCl solution (Ringer solution) and were maintained at 37°C for different time periods. Compressive strength was measured using a universal mechanical testing machine with a 10 kN load cell and clamp displacement speed of 1 mm/min. Compressive strength of the cements at different setting times is depicted in Figure 1.

Figure 2 depicts the X-ray diffraction diagram of the set cement for 7 days. The diffraction maximums observed correspond to magnesium oxide, which is the reagent present in excess in the formulation. This indicates that an amorphous compound is formed during the setting of the cement.

### Example 2: Evolution of the pH produced by a magnesium sodium phosphate powder.

Calcined MgO powder with a particle size between 0.1 and 40 µm and a specific surface area of 0.63 m²/g, and NaH₂PO₄ powder with a particle size between 100 and 500 µm and a specific surface area of 0.07 m²/g were mixed with a MgO:NaH₂PO₄ molar ratio of 3.8:1.

A saturated solution of the cement was prepared by mixing the powder mentioned in the preceding paragraph with water at a liquid/powder ratio of 10 mL/g, and the evolution of pH over time was measured. Said process was reported by Serraj [12] as an indirect method for evaluating the antimicrobial effect of a cement due to the basicity produced in its environment.

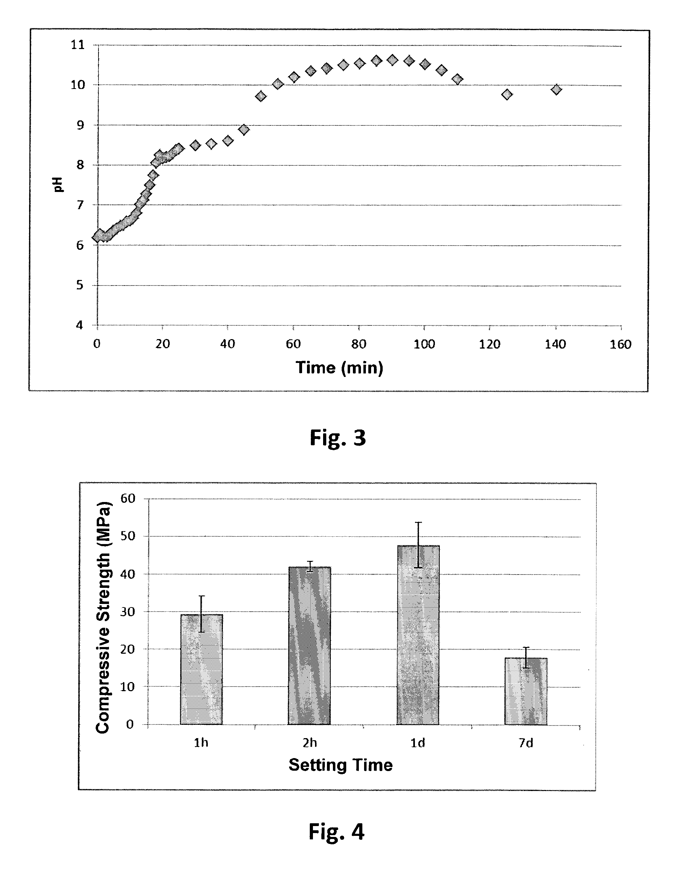

Figure 3 shows how a saturated solution of magnesium sodium phosphate powder makes pH rapidly increase, giving pH close to 9.5 in only 20 min and reaching pH greater than 10.5 in 90 min. It is known that microbes are sensitive to pH values higher than 9.5, accordingly this pH is sufficient for having an antimicrobial effect.

### Example 3: Magnesium phosphate cement prepared based on magnesium oxide, sodium hydrogen phosphate, ammonium hydrogen phosphate and borax

50 g of MgO calcined at 1475°C for 6 h were ground in an agate jar by means of using 4 agate balls, a planetary mill and grinding conditions of 150 rpm for 15 min. The same process was performed separately for NH₄H₂PO₄, NaH₂PO₄ and borax. 25 g of MgO were mixed with 9.388 g of NH₄H₂PO₄, 9.792 g of NaH₂PO₄ and 1.484 g of borax in a homogeniser for 20 min. The preparation of the paste consisted of mixing 1.5 g of powder with 195 µL of water, and it was homogenised for 1 min to yield a paste with good consistency.

The initial and final setting time was measured by the Gillmore needle test as described in Example 1. The test was performed by introducing the cement paste into plastic cylindrical moulds 10 mm in height. The initial setting time of the cement was 11.5 min, and the final time was 13 min.

The exothermic nature of the cement was evaluated by inserting the tip of a thermocouple into the paste. The temperature of the cement was monitored until it reached its maximum value. The maximum temperature reached during the setting of the cement was 44°C.

Test specimens of the cement 12 mm in height and 6 mm in diameter were prepared in Teflon moulds to measure compressive strength. Said moulds were introduced in a 0.9% wt NaCl solution (Ringer solution) and were maintained at 37°C for different time periods. Compressive strength was measured using a universal mechanical testing machine with a 10 kN load cell and clamp displacement speed of 1 mm/min. Compressive strength of the cements at different setting times is depicted in Figure 4.

### Brief Description of the Figures

The following has been depicted in the figures.

Figure 1 shows the compressive strength of magnesium sodium phosphate cement at different setting times (1 h = 1 hour; 2 h = 2 hours; 1 d = 1 day; 7 d = 7 days).

Figure 2 shows X-ray diffraction of the reaction product of set magnesium sodium phosphate cement in Ringer's solution for 7 days.

Figure 3 shows a saturated solution of the magnesium sodium phosphate powder in water with a liquid/powder ratio of 10.

Figure 4 shows the compressive strength of magnesium sodium and ammonium phosphate cement at different setting times (1 h = 1 hour; 2 h = 2 hours; 1 d = 1 day; 7 d = 7 days).

### Invention Scalable to Industrial Application

The magnesium sodium phosphate cement which has been described has several applications in the clinical field, especially in the fields of orthopaedic surgery, endodontics, the prosthodontics and periodontics. This invention can be easily applied at an industrial scale. Preparing the powder of the cement is very simple since all the reagents are commercial reagents and it only requires optimising reactivity for being able to control the properties listed below, which increase the value of said cement in the field of biomaterials:
(a) Increase of the pH of the medium surrounding the cement, giving it an antimicrobial effect;
(b) Very fast setting accompanied by high compressive strengths;
(c) Controlled exothermic nature to prevent tissue necrosis;
(d) No release of toxic by-products;
(e) Adherent to living materials or objects;

Preparing the cement is also simple. The use of the cement in biomedical applications requires sterilisation of the reagents as well as an adequate use thereof; likewise, the cement must be introduced into the bone or dental defect in a short time period, limited by its hardening.

### LITERATURE

[1] Dorozhkin SV. Calcium orthophosphate cements for biomedical application. Journal of Materials Science 43 (2008) 3028-3057.
[2] Wilson AD, Nicholson JW. Acid-base Cements: Their Biomedical and Industrial Applications. Chemistry of Solid State Materials 3(1993). ISBN 0-521-37222-4.
[3] Stierli R.F, Tarver CC, Gaidis JM. Magnesium phosphate concrete. US Patent 3,960,580 (1976)
[4] Sherif FG, Ciamei AG. Fast-setting cements from superphosphoric acid. US Patent 4,734,133(1988)
[5] Wu F, Wei J, Guo H, Chen F, Hong H, Liu C. Self-setting bioactive calcium-magnesium phosphate cement with high strength and degradability for bone regeneration. Acta Biomaterialia 4 (2008) 1873-1884.
[6] Michalowski T, Pietrzyk A. A thermodynamic study of struvite + water system. Talanta 68 (2006) 594-601.
[7] Liu C. Inorganic bone adhesion agent and its use in human hard tissue repair. US Patent 7,094,286 B2 (2006).
[8] Zimmermann M. Magnesium-ammonium-phosphates cements, the production of the same and the use thereof. US Patent 6,692,563 B2 (2004).
[9] Zimmermann M. Magnesium ammonium phosphate cement composition. US Patent 7,115,163 B2 (2006).
[10] Lally T. Bio-adhesive composition, method for adhering objects to bone. US Patent 6,533,821 B1.
[11] Klammert U, Reuther T, Blank M, Reske I, Barralet JE, Grover LM, Kübler AC, Gbureck U. Phase composition, mechanical performance and in vitro biocompatibility of hydraulic setting calcium magnesium phosphate cement. Acta Biomaterialia, doi: 10.1016/j.actbio.2009.10.021
[12] Serraj S, Michaïlesco P, Margerit J, Bernard B, Boudeville P. Study of a hydraulic calcium phosphate cement for dental applications. Journal of Material Science: Materials in Medicine 13 (2002) 125-131.
[13] Gbureck U, Knappe O, Grover LM, Barralet JE. Antimicrobial potency of alkali ion substituted calcium phosphate cements. Biomaterials 26 (2005) 6880-6886.
[14] Gbureck U, Knappe O, Hofmann N, Barralet JE. Antimicrobial properties of nanocrystalline tetracalcium phosphate cements. J Biomed Mater Res Part B: Appl Biomater 83B (2007) 132-137
[15] Sawai J, Kojima H, Igarashi H, Hashimoto A, Shoji S, Sawaki T, Hakoda A, Kawada E, Kokugan T, Shimizu M. Antibacterial characteristics of magnesium oxide powder. World Journal of Microbiology & Biotechnology 16 (2000) 187-194.
[16] El-Jazairi B. Rapid repair of concrete pavings. Concrete 16 (1982) 12-15.
[17] Soudée E, Péra J. Mechanism of setting reaction in magnesia-phosphate cements. Cement and Concrete Research 30 (2000) 315-321.
[18] ASTM-Standard C 266 - 03: Test Method for Time of Setting of Hydraulic-Cement Paste by Gillmore Needles, ASTM. International 2003.

## Claims

1. An inorganic cement for biomedical applications comprising a mixture of a solid phase formed by magnesium oxide (MgO) and a sodium phosphate, and a liquid phase formed by water or an aqueous solution.

2. The cement according to claim 1, **characterised in that** the mixture reaction product is amorphous and increases the pH of the surrounding medium, giving it antimicrobial properties.

3. The cement according to claim 1, wherein the sodium phosphate is sodium dihydrogen phosphate (NaH₂PO₄).

4. The cement according to claim 3, **characterised in that** the magnesium oxide to sodium phosphate (MgO:NaH₂PO₄) molar ratio is greater than 1.

5. The cement according to claim 4, wherein the magnesium oxide to sodium phosphate molar ratio is in the 1<MgO:NaH₂PO₄<6 range, the 3<MgO:NaH2PO4<5 range being preferred.

6. The cement according to claim 1, **characterised in that** it comprises a retarder from among: sodium borate, boric acid, boric acid ester, sodium fluorosilicate, sodium polyphosphate and mixtures derived therefrom, at an amount of 0.05-10% by weight with respect to the powder phase of the cement, an amount between 2-6% by weight being more preferable.

7. A method for preparing an inorganic cement for biomedical applications according to claim 1, **characterised in that** the MgO is calcined at a temperature greater than 1400°C for 0.5-15 h for the purpose of reducing its reactivity.

8. The method according to claim 7, wherein the setting time and the exothermic nature of the reaction are controlled by means of adding a retarder in the solid phase or in the liquid phase of the cement.

9. The method according to claim 8, **characterised by** using sodium borate, boric acid, boric acid ester, sodium fluorosilicate, sodium polyphosphate and mixtures derived therefrom as a retarder, out of which sodium borate decahydrate or borax is preferred.

10. The method according to claim 7, wherein the retarder is added at an amount of 0.05-10% by weight with respect to the powder phase of the cement, adding between 2-6% by weight being more preferable.

11. Use of a cement according to any one of claims 1 to 6 for bone and dental applications, especially in situations in which fighting a microbial infection is desired.

12. Use of a cement according to any of claims 1 to 6 for endodontic treatments such as directly and indirectly covering the pulp, sealing dentinal tubules, treating periapical inflammations or generally carrying out endodontic or apexification therapies.
